# EUROPEAN PATENT SPECIFICATION

(11) **EP 3 302 261 B1**
(45) Date of publication and mention of the grant of the patent: **13.01.2021**
(21) Application number: 16804435.2
(22) Date of filing: 02.06.2016
(51) Int. Cl.: A61B 5/05, G01R 33/00, G01R 33/02

(54) **MAGNETIC SENSING TO PROVIDE GEOMETRY INFORMATION**
MAGNETISCHE MESSUNG ZUR BEREITSTELLUNG VON GEOMETRIEDATEN
DÉTECTION MAGNÉTIQUE POUR FOURNIR DES INFORMATIONS DE GÉOMÉTRIE

(30) Priority: 02.06.2015 US 201562169896 P
(43) Date of publication of application: 11.04.2018
(73) Proprietor: CardioInsight Technologies, Inc., Independence, OH 44131 (US)
(72) Inventor: ZENG, Qingguo, Solon, OH 44139 (US); JIA, Ping, Solon, OH 44139 (US); RAMANATHAN, Charulatha, Solon, OH 44139 (US); LOU, Qing, Powell, OH 43065 (US); BOKAN, Ryan, Independence, OH 44131 (US); GEORGE, Brian P., Cleveland, OH 44111 (US)
(74) Representative: Schmidt, Steffen J.
(86) International application number: PCT/US2016/035516
(87) International publication number: WO 2016/196793

(56) References cited:
- EP-B1- 1 125 549
- US-A1- 2009 227 840
- US-A1- 2012 004 540
- US-A1- 2014 206 985
- US-A1- 2015 073 268
- US-B2- 7 983 743

## Description

### CROSS-REFERENCE TO RELATED APPLICATION

This application claims the benefit of U.S. provisional patent application no. 62/169,896 filed on June 2, 2015 and entitled USING MAGNETIC SENSORS TO CREATE GEOMETRIES AND NAVIGATE CATHETERS,.

### TECHNICAL FIELD

This disclosure relates to using magnetic sensors to provide geometry information.

### BACKGROUND

This section provides background information related to the present disclosure and is not necessarily prior art.

Noninvasive electrocardiographic imaging involves inverse reconstruction of electrical potentials sensed on a body surface geometry to another surface geometry within the body. The inverse reconstruction utilizes geometry information that is typically derived from imaging data, such as from magnetic resonance imaging (MRI), computed tomography (CT) or another imaging modality. In some cases, such imaging can be expensive and/or difficult to coordinate with cardiac electrophysiology studies.

US 2012/0004540 A1 relates to a field positioning equipment for creating a three-dimensional model for a heart cavity inner wall of a patient. The equipment comprises a magnetic field generating device which generates a plurality of distinguishable fields under excitation of a drive signal and at least one magnetic field sensor located in a distal end portion of the heart cavity for detecting the distinguishable fields to generate a sensor signal. The equipment further comprises a positioning signal processing device for transmitting the drive signal to the magnetic field generating device and detecting the output of the magnetic field sensor signal to acquire data of five-dimensional position and orientation coordinates of a position on the distal end portion of the heart cavity where the magnetic sensor is located. The equipment further comprises a body surface reference positioning electrode which is mounted on the patient's body surface and provides a reference point. The distal end portion of the reference electrode is mounted with at least two micro electromagnetic field sensors.

US 2009/0227840 A1 relates to a capsule endoscope guiding system. The guiding system comprises a magnetic field generating device generating a three-dimensional rotating magnetic field. Further, the system comprises electrode pads to perform human body communication with a capsule endoscope and detect position and direction of the capsule endoscope. The electrode pads are provided on a surface of the human body. A magnetic sensor is attached to an outside surface of each electrode pad. The magnetic sensors detect the three-dimensional position of each electrode pad.

### SUMMARY

This disclosure relates to using magnetic sensors to provide geometry information.

As one example, a system includes an electromagnetic spatial measurement apparatus. The measurement apparatus includes: a moveable sensor to provide a sensor signal in response to an electromagnetic field, and a plurality of stationary sensors on an outer surface of a patient's body that each provides respective sensor signals in response to the electromagnetic field. The measurement apparatus provides first position data representing multiple positions of the moveable sensor in a given coordinate system. The measurement apparatus also provides second position data based on a position of each of the plurality of sensors in the given coordinate system. The system further comprises a plurality of electrodes on the outer surface of the patient's body to sense electrical activity from locations on the outer surface of the patient's body. The system also includes a processor configured to:
compute internal geometry data based on the first position data, the internal geometry data representing a plurality of locations in a three-dimensional space distributed across an anatomical surface within the patient's body;
compute electrode geometry data based on the second position data, the electrode geometry data representing the location of each of the plurality of electrodes on the outer surface of the patient's body; and
reconstruct cardiac electrical activity onto a cardiac envelope based on the sensed electrical activity from the outer surface of the patient's body, the internal geometry data and the electrode geometry data.

As another example (not comprised by the claims), a method includes storing invasive position data representing different positions of one or more sensors in a given coordinate system within a volume defined by an electromagnetic field and storing non-invasive position data representing different positions of a plurality of control points in the given coordinate system determined from a position of one or more sensors. The method also includes computing internal geometry data based on the invasive position data, the internal geometry data representing a three-dimensional anatomical surface within a patient's body. The method also includes computing electrode geometry data based on the non-invasive position data, the electrode geometry data representing a location of each of a plurality of electrodes on an outer surface of the patient's body. Electrical activity sensed by the plurality of electrodes can be reconstructed onto an anatomical envelope within the patient's body based on the internal geometry data and electrode geometry data.

### BRIEF DESCRIPTION OF THE DRAWINGS

FIG. 1 depicts an example of a system to provide geometry data using magnetic sensors.
FIG. 2 depicts an example of a diagnostic/treatment system that utilizes electromagnetic sensors to determine geometry information to enable electrogram reconstruction.
FIG. 3 depicts an example of a diagnostic/treatment system that utilizes electromagnetic sensors to determine geometry information to enable electrogram reconstruction based on invasive and/or noninvasive electrical measurements.
FIG. 4 depicts an example of a sensor apparatus that includes electrical and electromagnetic sensors.
FIG. 5 depicts part of an example catheter device that includes electromagnetic sensor and one or more sensor electrodes.
FIG. 6 depicts an example system to generate a domain translator to facilitate localization based on the electrical sensor signals.
FIG. 7 depicts an example of a diagnostic/treatment system that can utilize a domain translator of FIG. 6 to enable localization and/or navigation.
FIG. 8 is a flow diagram depicting an example of a method for determining geometry information such as for electrogram reconstruction.
FIG. 9 is a flow diagram depicting an example of a method for localizing an object in a patient's body.

### DETAILED DESCRIPTION

This disclosure provides systems and methods that utilize magnetic sensors to generate geometry information. The geometry information created using the magnetic sensors can be utilized to perform electrogram reconstruction, in which electrograms are reconstructed onto an anatomical envelope within a patient's body, such as a cardiac surface (e.g., the epicardial or endocardial surface) or another surface within the patient's body. Additionally or alternatively, the geometry information that is generated can be utilized to help localize and/or navigate in an implantable device relative to the anatomical structures.

As a further example, one or more EM sensors (also referred to herein as a receiver) may be movable within a patient's body while within an EM field provided by an EM field generator (also referred to as a transmitter). For instance, the sensor(s) can be attached to or integrated into a probe or catheter within the EM field and provide a sensor signal corresponding to at least a location thereof in a three-dimensional coordinate system. The probe or catheter thus can be moved to a plurality of different locations to capture sensor signals for a plurality of locations across a region of interest, which locations can be combined to generate corresponding geometry data representing for the region of interest. One or more magnetic sensors may also be provided at fixed locations within the patient's body (e.g., one or more heart locations, such as sinoatrial node, or other anatomical landmarks). As an example, the geometry can include an anatomical geometry, such as the heart surface, (e.g., epicardial and/or endocardial surface) or another three-dimensional construct within the body.

In some examples, the probe with the magnetic sensor can be utilized as a digitizer that is moved into engagement to detect the location of one or more electrical sensors that may be disposed at stationary (e.g., fixed) locations non-invasively on a patient's body surface. In other examples, one or more magnetic sensors may be integrated into or otherwise co-located with some or all of the electrical sensors that are distributed across the body surface. Since the magnetic sensors have a known (predetermined) position with respect to such electrical sensors, the locations of the magnetic sensors can be used to ascertain geometry data representing the location of the body surface electrical sensors in a common coordinate system with the geometry for the invasive region of interest.

The resulting geometry information can be utilized for forward and/or inverse calculations for reconstructing electrical activity, including electrical cardiac signals from the heart, electrical signals measured from body surface electrodes, electrical signals measured from a catheter that may be moved within a patient's body as well as any combination thereof. Additionally or alternatively, the geometry information can be used to facilitate localization and navigation of objects within the patient's body.

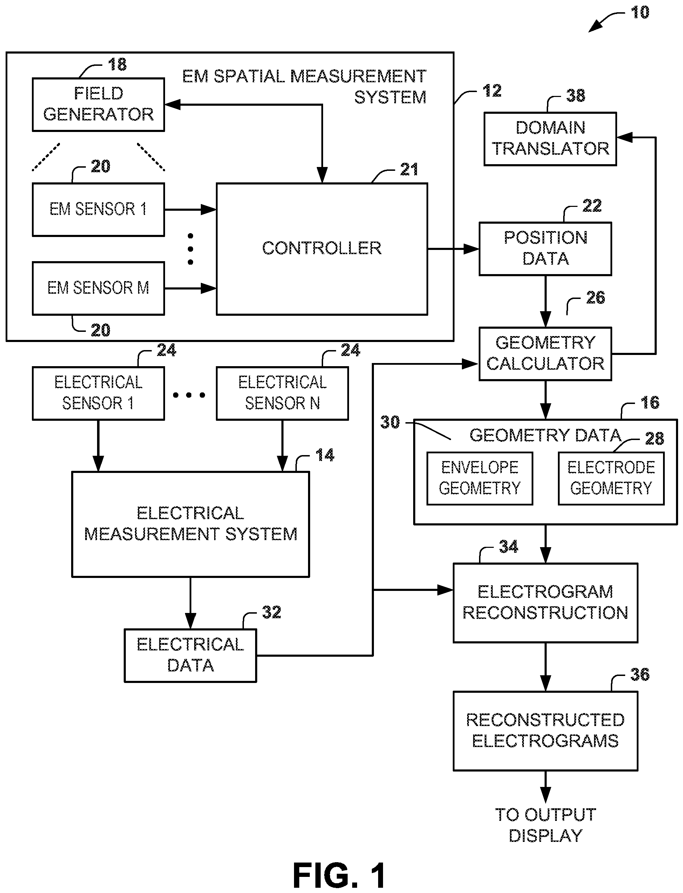

FIG. 1 depicts an example of a system 10 to generate geometry information that can be utilized to facilitate electrogram reconstruction, such as disclosed herein. The system 10 includes both an electromagnetic (EM) spatial measurement system 12 and an electrical measurement system 14. The EM spatial measurement system 12 is utilized to generate geometry data 16 without requiring imaging data as in prior approaches. Instead, as disclosed herein, the EM spatial measurement system 12 determines locations of EM sensors 20 associated with an invasive region of interest within the patient's body and locations of EM sensors associated with electrode locations on the patient's body surface.

In the example of FIG. 1, the EM measurement system 12 includes a field generator 18 that generates an EM field that defines a volume interest. The EM measurement system 12 also includes plurality of sensors 20, demonstrated as EM sensor 1 through EM sensor M, where M is a positive integer. Each sensor 20 thus generates a respective sensor signal in response to the EM field, such as an alternating EM field. The sensor signals are provided to a controller 21. The controller 21 can include interfaces to amplify and filter the sensor signals. The controller 21 are includes hardware and/or software (e.g., instructions executable by a processor) to determine compute position of each sensor and provide position data 22 corresponding to a three-dimensional location of each respective sensor within the EM field. The controller 21 can also control the field generator 18 to provide EM field. As an example, the EM spatial measurement system 12 can be similar to or correspond to the Aurora® EM tracking system available from Northern Digital Inc.

By way of example, one or more of the EM sensor(s) 20 can be moveable within the alternating EM field, and the field can induce electrical current in the sensor. The induced signal can vary depending on the location and orientation of the sensor with respect to the field. As an example, one or more EM sensors 20 are fixed to part of a probe, such as a catheter or other guide mechanism (e.g., guide wire). For instance, the probe can be inserted into the patient's body in a minimally invasive manner and moved to a plurality of locations across an anatomical surface within the patient's body and the sensor signals can be used to generate position data 22 corresponding to an anatomical surface region of interest. The region of interest thus corresponds to surface region inside the body and the locations across such surface can be recorded in the coordinate system of the EM measurement system 12 and stored in memory as part of the corresponding position data 22 to provide a plurality of control points residing on the anatomical surface region of interest.

As an example, a vascular catheter can be fitted with one or more electromagnetic tracking sensors having with three, four, five or six degrees of freedom. A five degree of freedom EM tracking sensor enables tracking of x, y, and z spatial coordinates, as well as angular coordinates characterizing rotation about the transverse axis (pitch) and anterior-posterior axis (roll). A six degree of freedom sensor affords an additional capacity for tracking angular coordinates characterizing rotation about the inferior-superior axis (yaw). According to the examples provided in this disclosure, such as for purposes of mapping position, it is sufficient for the EM spatial measurement system 12 to employ sensors to provide the position data 22 including three-dimensional spatial coordinates.

As an example, one or more of the sensors 20 can be movable within the EM field such that the position data 22 associated with each such movable sensor can represent multiple positions of each such sensor within the three-dimensional coordinate system. For example, the field generator 18 provides an EM field within a three-dimensional volume of interest, which can be positioned near a patient's body to generate the corresponding position data 22 based on positioned determined for EM sensors located within the field volume. The position data thus is associated with the patient's body and can correspond specifically to locations distributed across one or more regions of interest within a patient's body. For instance, the sensor 20 can be moved across the surface of the patient's heart and corresponding points distributed across the surface can be obtained over time to generate the corresponding position data 22 by the measurement system 12. In some examples, the movement of the probe carrying the sensor 20 as may be guided by an imaging modality, such as ultrasound or fluoroscopy, during the positioning of the probe (e.g., catheter) containing one or more sensors 20. Alternatively, visual guidance can be omitted.

In some examples, one or more EM sensors 20 can be fixed at a known anatomical position within the patient's body to provide the corresponding system signal associated with the fixed position in response to the EM field. The sensor at the fixed known anatomical position thus can be utilized as a control point to facilitate determining geometry from the other sensor locations within the coordinate system.

Additionally, one or more of the EM sensors 20 further can be utilized outside the patient's body to ascertain locations of electrical sensors located outside of the patient's body or otherwise distributed around the invasive locations determined within the patient's body. The non-invasive EM sensors 20 can have fixed locations with respect to the electrical sensors 24, such as connected to the electrical sensors or to a common substrate to which both the EM and electrical sensors are mounted. Such non-invasive EM sensor 20, for example, can be located at predetermined control points outside the patient's body having a known spatial location with respect to one or more of the non-invasive electrical sensors 24. Some or all such stationary non-invasive EM sensors 20 can be co-located (e.g., overlying relationship) with respect to the electrical sensors 24. As another example, one or more non-invasive EM sensors 20 may be moveable into contact with the electrical sensors, such as part of a digitizer probe, and when contact is made the resulting position data 22 can be tagged to identify contact with a respective electrical sensor. Regardless of being stationary or moveable, the field generator 18 induces a sensor signal in each of the EM sensors having a signal strength that depends on its location (spatial coordinates) within the EM field, which location is recorded in the position data generated by the controller 21.

In the example of FIG. 1, the system 10 includes a plurality of electrical sensors, demonstrated at electrical sensor 1 through electrical sensor N, where N is a positive integer greater than 2. For example, N can be virtually any number, such as 50, 100, 200, 252 or other numbers of electrodes. In some examples, the electrical sensors can be implemented as part of a wearable garment or patch containing the electrodes attached to a corresponding substrate that maybe affixed to a patient's body, such as along and around the patient's thorax. In this way, electrical sensors 24 can be distributed substantially evenly or in another desired arrangement around and attached ergonomically to the patient's body. A set of the EM sensors 20 can be positioned on the same substrate as at a set of the electrical sensors 24. For example, EM sensors 20 can be positioned at corresponding control points having a predetermined location with respect to each the locations of the electrical sensors 24 sufficient to ascertain the locations of the electrical sensors from locations of the EM sensors.

As one example, the electrical sensors 24 can be implemented as an arrangement of sensors to cover a preselected portion of the patient's body surface, such as disclosed in U.S. Patent Publication No. 2013/0281814, entitled MULTI-LAYERED SENSOR APPARATUS; although other configurations and arrangements of electrical sensors 24 could be utilized. As another example, the arrangement of sensors can be implemented as set or zone of electrodes configured to cover selected parts of the patient's body surface that map deterministically to a given region or interest on one or more surface areas of the heart, such as disclosed in U.S. Patent Publication No. 2016/0067489, entitled SYSTEM AND METHODS TO FACILITATE PROVIDING THERAPY TO A PATIENT.

The geometry calculator 26 computes the envelope geometry 30 based upon the position data 22 collected by one or more EM sensors 20. By moving such EM sensors within the patient's body move across the surface region of interest, the geometry calculator 26 thus can compute the envelope geometry 30 to correspond to an actual surface of the patient's heart (e.g., epicardial surface and endocardial surface). In other examples, the geometry calculator 26 computes the envelope geometry to correspond to a virtual surface having a predetermined geometrical relationship with respect to the patient's heart according to the locations on the region of interest that have been tagged during the mapping by the movable EM sensor 20. As mentioned, the region of interest can correspond to a predetermined contiguous surface area of a patient's heart, a plurality of separate regions across the patient's heart, or the entire heart surface. Since the movable EM sensor 20 can be moved and engaged with the patient's anatomy, the mapping process can be repeated as the EM sensor 20 tags a plurality of spaced apart locations distributed across the surface of interest to create an accurate representation of organ geometry (e.g., endocardial chambers, epicardial surface, esophagus and the like). The tagging of spaced apart locations across the surface can be manual in response to a user input (e.g., activation of a trigger or button) or it can be an automated process by which position data is processed to tag a designated number of points within a surface area (e.g., a predetermined density of tagged locations per surface area). The geometry calculator 26 computes the envelope geometry 30, for example, to define a mesh surface of the envelope corresponding to the region of interest (e.g., a cardiac mesh).

Additionally, the geometry calculator 26 can determine electrode geometry 28 in a corresponding three-dimensional coordinate system based on position data 22 describing spatial coordinates for a set of EM sensor locations associated with the electrical sensors 24. As mentioned, a set of non-invasive EM sensors 20 can be located at predetermined control points outside the patient's body having a known spatial location with respect to one or more of body surface electrical sensors 24. Alternatively, one or more non-invasive EM sensors 20 may be moveable into contact with the electrical sensors, such as part of a digitizer probe, and when contact is made the resulting position data 22 can be tagged to identify contact with a respective electrical sensor. The geometry calculator 26 thus computes electrode geometry 28 from the associated position data, such as to represent centers (e.g., centroids) of each sensor electrode that is positioned on body surface. Once calculated, the envelope geometry 30 and electrode geometry 28 are stored in memory (e.g., one or more non-transitory machine readable media) to facilitate electrogram reconstruction and/or localization based on measured body surface electrical activity. In some examples, the electrogram reconstruction and/or localization may be performed based on measured body surface electrical activity and stored geometry data, even in the absence of further use of the EM spatial measurement system.

The electrical measurement system 14 can receive sensor signals from each of the electrical sensors 24 to provide corresponding electrical data 32 for one or more time intervals. The electrical data 32 thus can correspond to electrical signals measured at a plurality of locations distributed across the patient's body where the electrical sensors 24 are located. The locations of the sensors can be computed by the geometry calculator corresponding to the electrode geometry 28, such as disclosed herein. The electrical data 32 thus can include data representing the time-varying amplitude of the measured electrical potential, which can be time-stamped corresponding to the time associated with the amplitude that varies as a function of time.

The system 10 can also include electrogram reconstruction function 34 that is programmed to generate reconstructed electrograms 36 based on the electrical data 32 and the geometry data 16. For example, the electrogram reconstruction function 34 can solve the inverse problem by reconstructing electrical activity for each of the electrodes according to the electrode geometry 28 onto a corresponding cardiac envelope based on the envelope geometry 30. Examples of solutions to the inverse problem include the boundary element method (BEM) or the method of fundamental solution (MFS). The reconstructed electrograms thus can correspond to electrograms across an envelope, and can include static (three-dimensional at a given instant in time) and/or be dynamic (e.g., four-dimensional map that varies over time) based on the measured electrical data 32.

Examples of inverse algorithms that can be utilized in the system 10 include those disclosed in U.S. Patent Nos. 7,983,743 and 6,772,004. The EGM reconstruction 186 thus can reconstruct the body surface electrical activity measured via the sensors 164 onto a multitude of locations on an envelope (e.g., greater than 1000 locations, such as about 2000 locations or more). In other examples, the processing system 162 can compute electrical activity over a sub-region of the heart based on electrical activity measured invasively, such as via the device 156 (e.g., including a basket catheter or other form of measurement probe). The resulting reconstructed electrograms 36 can be stored in memory and provided as an output to the display such as to provide electrocardiographic maps of electrical potentials or other signal characteristics that can be derived from the reconstructed electrograms on the cardiac envelope.

In some examples, the position data 22 can also include a time-stamp that is synchronized temporally with respect to the measured electrical data 32. By synchronizing the position data 22 with the electrical data 32, a probe (e.g., catheter) carrying an electrode can be utilized to supply an electrical signal via the electrode at a spatial location within the patient's body and the supplied signal can be detected via the arrangement of body surface electrical sensors 24. The electrical signal that is applied to the electrode on the movable catheter, for example, can be a pulse wave, a square wave, a sinusoidal wave, a triangle wave or other predetermined waveform, which may be periodic or supplied intermittently such as in response to a user input. Thus, by collecting position data 22 and electrical data 32 synchronized in time for a given known position where an electrical signal is supplied via the electrode, the electrical data can be registered with respect to the position data 22 (e.g., by translator generator of the geometry calculator 26) to generate a domain translator 38. The domain translator 38 thus can be used translate sensed electrical information to position information in a given coordinate system, which may be the same or a different coordinate system from the EM measurement system 12.

As one example, the domain translator 38 can be in the form of a look-up table (LUT) in which the sensor signals for each of the plurality of electrical sensors 24 operate as an index to a given position derived from the position data at a corresponding location within the patient's body where the signal was applied. By supplying signals at a plurality of different locations distributed across a region of interest, a look up table for mapping between the sensed electrical data and spatial coordinates can be generated for the region of interest. In this way, by a user positioning a catheter, which may or may not include an EM sensor, at a location on the surface that has been mapped in this manner and supplying an electrical signal (e.g., the same signal that was utilized to generate the look up table), the look-up table 38 can provide a location estimate according to which location best matches the sensed electrical signals provided in the look-up table. As a further example, the spatial location can be estimated by the LUT interpolating among multiple entries according to the measured electrical signals (sensed by the electrical sensors 24) in response to the signal that is applied to the electrode on the catheter within the patient's body. By determining position over time, movement of the catheter and its trajectory can also be ascertained by the domain translator LUT.

As another example, the domain translator 38 can be implemented as a transform that converts measured electrical signals into spatial coordinates in a given coordinate system. The transform can be generated through a mapping and registration process in which the sensed electrical signals by creating respective geometries using a localization process (e.g., dipole localization) in a first spatial domain and relative positions between the magnetic sensor locations determined for a second spatial domain. A transform generation process can be utilized to convert position data from the first spatial domain, as determined via the localization process, into the second spatial domain corresponding to the EM measurement system. For example, a catheter can be positioned at a location within a region of interest that was utilized in creating the transform, and the electrical data provided by the electrical sensors 24 in response to the signal applied to the electrode can be localized into coordinates in the first spatial domain. The transform is then applied to the coordinates in the first spatial domain to provide a corresponding spatial location associated with the catheter in the second spatial domain. Thus, the transform can provide a dynamic mapping between the spatial domain of the magnetic sensors locations that were detected during the mapping and calibration process and a different domain associated with the electrode locations via another localization process.

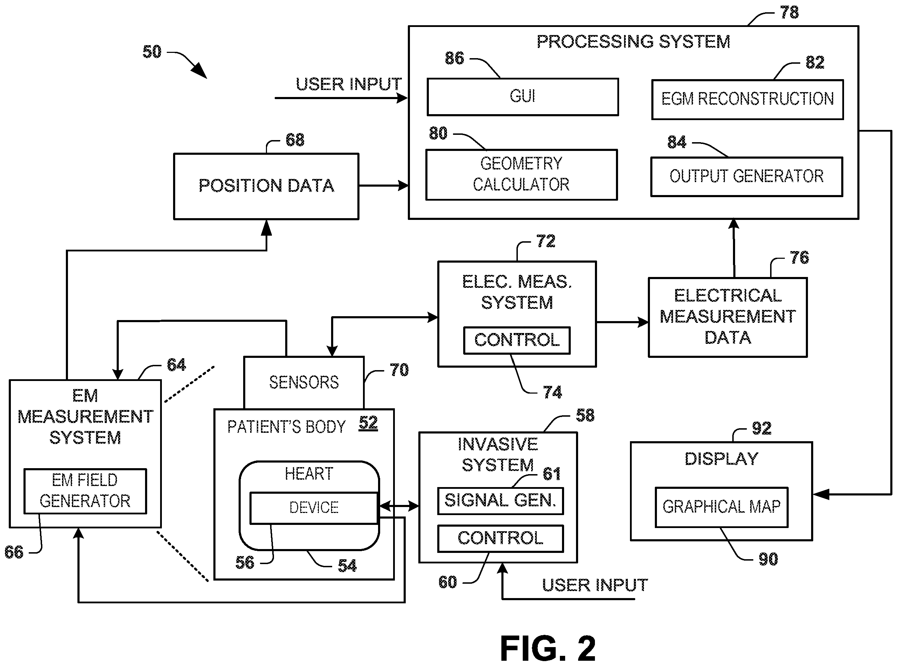

FIG. 2 depicts an example of a system 50 to facilitate diagnosing and/or treating a region of interest within a patient's body 54. In some examples, the system 50 can be implemented to generate corresponding graphical outputs for signals and/or graphical maps for a patient's heart 52, including reconstructed electrograms, localization of an object, in real time as part of a procedure (e.g., monitoring of signals during an electrophysiology study). Additionally or alternatively, the system 50 can be utilized as part of a treatment procedure, such as to help guide a physician to deliver a therapy to a desired target site or region.

For example, a device 56, such as a catheter, can be inserted into a patient's body 54, such as through a low or minimally invasive procedure. The device 56 includes one or more electrodes configured to deliver energy and/or sense electrical activity within the patient's body 54. The device 56 can apply energy via one or more electrodes as a localization-specific signal, a pacing signal or to deliver another therapy, such as to electrically affect tissue (e.g., providing electrical therapy, or controlling delivery of chemical therapy, sound wave therapy, thermal therapy or any combination thereof). The device 56 can be inserted within or near the heart 52 to sense and/or deliver energy in a contact or non-contact manner. Additionally or alternatively, the device 56 can include one or more EM sensors that provide sensor signals to an EM spatial measurement system 64 in response to an EM field (e.g., sensor 20 of FIG. 1). The placement of the device 56 can be guided via a localization method (e.g., via EM measurement system 64 or other) and/or intraprocedural imaging modality (e.g., x-ray fluoroscopy, ultrasound, CT or the like). The guidance can be automated, semi-automated or be manually implemented based on information provided.

An invasive system 58 can include a control 60 configured to control a signal generator 61 to apply a signal via one or more electrodes of the device 56. For example, the control 160 can control parameters (e.g., current, voltage, repetition rate, trigger delay, sensing trigger amplitude) of the signal generator 61 for delivering therapy (e.g., ablation or stimulation) via the electrode(s) to one or more location of the heart 52. The control 60 can set the therapy parameters and apply stimulation based on automatic, manual (e.g., user input) or a combination of automatic and manual (e.g., semiautomatic) controls. For example, the invasive system 58 can be located external to the patient's body 54 and be configured to control delivery of electrical signals via one or more electrodes of the device 56. For instance, the system 58 can also control electrical signals provided via a conductive link electrically connected between the delivery device (e.g., one or more electrodes) 56 and the system 58. One or more sensors (not shown but could be part of the device 56) can also communicate sensor information to a processing system 78.

The EM spatial measurement system 64 can be implemented as corresponding to the EM spatial measurement system 12 of FIG. 1. Accordingly, reference can be made back to FIG. 1 for additional information and context. Briefly, the EM spatial measurement system 64 includes a field generator 66 that provides an alternating electromagnetic field to induce a signal of varying strength in each EM sensor. As mentioned, one or more EM sensor may reside on the invasive device 56 or another moveable probe. One or more EM sensors further can be positioned on the patient's body associated with electrical sensors 70 to provide respective sensor signals. The strength of each induced signal varies with the distance and relative orientation between the receiver and the field generator 66. Thus, the EM spatial measurement system 64 can derive the location of each EM sensor based on signal strength of the sensor signal and provide such location as corresponding position data 68.

Additionally, the system 50 includes a plurality of sensors 70 attached to the body 54 at locations. As disclosed herein, the EM measurement system 64 is used to ascertain electrode geometry that characterizes the location of each of the electrodes in given three-dimensional coordinate system. The sensors 70 thus can sense electrical activity on the body surface, including real-time electrical activity for the patient's heart 52 as well as electrical signals corresponding to the signals applied via the device as provided by the signal generator 61.

In the example of FIG. 2, one or more sensors 70 can be implemented as an array or other configuration for recording patient electrical activity. As one example, the sensors 70 can include to a high-density arrangement of body surface sensors that are distributed over a portion of the patient's torso for measuring electrical activity associated with the patient's heart (e.g., as part of an electrocardiographic mapping procedure), such as disclosed herein. Other arrangements and numbers of sensors 70 can be used. As another example, the sensors 70 can be a reduced set of sensors, which does not cover the patient's entire torso and is designed for measuring electrical activity for a particular purpose (e.g., an array of electrodes specially designed to map deterministically to a selected region of interest for analyzing AF and/or VF) and/or for monitoring a predetermined spatial region of the heart, as disclosed herein. A non-invasive electrical measurement system

As mentioned, the electrical sensors 70 provide real time sensed electrical signals to the electrical measurement system 72. The measurement system 72 can include appropriate control and signal processing circuitry 74 for providing corresponding measurement data 76 that describes electrical activity detected by the sensors 70. The measurement data 76 can include analog and/or digital information (e.g., corresponding to electrogram data 14). The control 74 can also be configured to control the data acquisition process (e.g., sample rate, line filtering) for measuring electrical activity and providing the measurement data 76. In some examples, the control 74 can control acquisition of measurement data 76 separately from the invasive system operation, such as in response to a user input. In other examples, the measurement data 76 can be acquired concurrently with and in synchronization with specific signals applied by the signal generator 61 for purposes calibrating and configuring a domain translator for localization. For instance, appropriate time stamps can be utilized for indexing the temporal relationship between the respective measurement data 72 and position data 68. In either example, the non-invasive measurement system 72 can measure the body surface electrical activity via the sensor to provide corresponding measurement data 76.

The processing system 78 thus can perform various signal processing and transformative methods, including a geometry calculator 80, electrogram reconstruction 82, a graphical user interface (GUI) 86 and an output generator 84 to control a display 92.

Since, in some examples, the measurement system 72 can measure electrical activity of a predetermined region or the entire heart concurrently (e.g., where the sensors 164 covers the entire thorax of the patient's body 154), the measurements are spatially and temporally consistent across the entire region of interest. Consequently, the accuracy in the resulting output location provided in the output data 174 can be increased when compared to other measurement approaches, such as to supply the user with a more accurate and global information to facilitate monitoring and application of therapy.

By way of further example, the geometry calculator is programmed to compute envelope geometry and electrode geometry, such as disclosed with respect to FIG. 1. Thus, the processing system 78 can store the computed envelope geometry and electrode geometry in memory to facilitate further processing, including electrogram reconstruction 82 based on the electrical measurement data. For example, the electrogram reconstruction 82 is programmed to compute an inverse solution and provide corresponding reconstructed electrograms based on the measurement data 76 and the geometry data computed by the geometry calculator 80, such as disclosed with respect to FIG. 1. The reconstructed electrograms thus can correspond to electrocardiographic activity across an envelope, and can include static (three-dimensional at a given instant in time) and/or be dynamic (e.g., four-dimensional map that varies over time).

As disclosed herein, the envelope geometry can represent to a three-dimensional surface geometry (e.g., a cardiac mesh) corresponding to a patient's heart, which surface can be epicardial or endocardial. In some examples, the geometry calculator 80 computes the envelope geometry to represent actual surface of the patient heart geometry based on EM position data 68 acquired during mapping by the catheter device 56. Alternatively, the geometry calculator 80 computes the envelope geometry based on the position data 68 to represent to a 3D geometric surface mesh that resides between the epicardial surface of a patient's heart and an outer surface of the patient's body where the electrical sensors 70 have been positioned. In addition to the envelope geometry, the geometry calculator also determines the electrode geometry as data representing the position of the electrodes in the sensor array in the same coordinate system as the envelope geometry, both of which can be stored in memory as geometry data. Appropriate anatomical or other landmarks, including locations for the EM or electrical sensors on or within the patient's body 54 can further be tagged and identified in the geometry data, such as for display in conjunction with other electrical or spatial information. The identification of such landmarks and can be done manually (e.g., by a person via image editing software) or automatically (e.g., via image processing techniques).

The output generator 188 can generate corresponding output data to provide a corresponding graphical map 90 in a display 92. For instance, the graphical map 90 can include reconstructed electrograms (e.g., a potential map) on the cardiac envelope. In some examples, a location for the device 56 is displayed on graphical model of patient anatomy or superimposed on the electrocardiographic map 90. The location can take other forms to provide guidance to the user, such as disclosed herein.

A graphical user interface (GUI) 86 can be employed to interact with the processing system 78 and/or the systems 72, 58 and/or 64. For example, the GUI 86 can be used to set parameters associated with the displayed graphical representation, corresponding to an output visualization of the computed map, such as including selecting a time interval, a type of information that is to be presented in the visualization and the like can be selected in response to a user input. Additionally, a user can employ the GUI 86 to selectively program one or more parameters (e.g., desired resolution, convergence thresholds, waveform parameters, spatial modeling and spatial thresholds, filter parameters and the like).

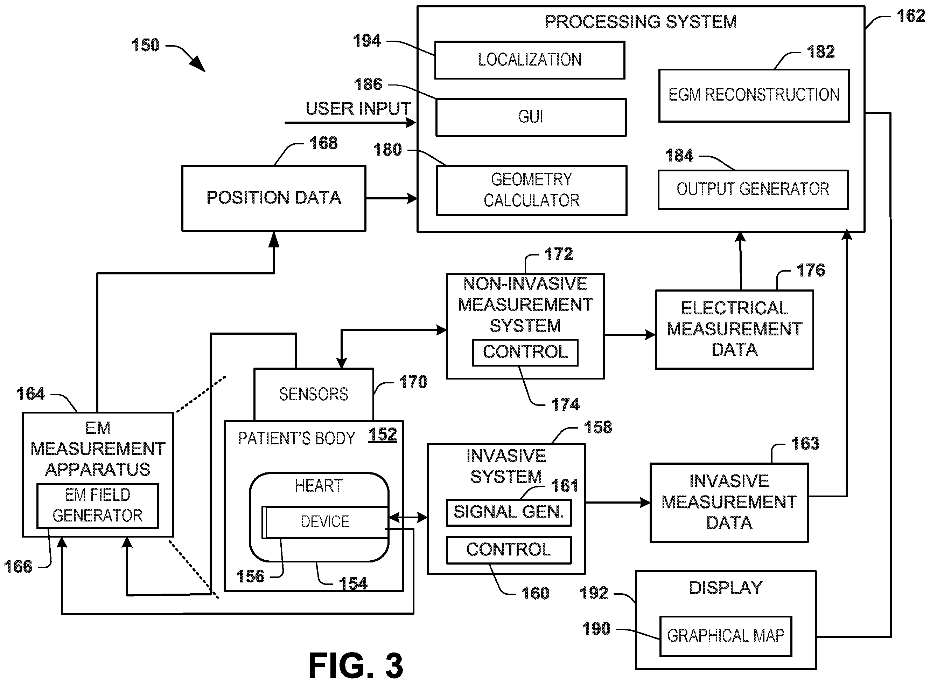

FIG. 3 depicts another example of a system 150 to facilitate evaluation and treatment of cardiac function. The same or similar components to those introduced with respect to FIG. 2 are demonstrated in FIG. 3 using the same reference numbers increased by adding 100. Accordingly, reference can be made back to the description of FIG. 2 for additional information about such common components.

In the example of FIG. 3, the invasive system 158 is further configured to measure electrical activity via one or more sensor electrodes on device 156. The invasive system thus employs the control and signal processing circuitry to amplify and filter sensed electrical activity and provide corresponding invasive measurement data 163. The invasive measurement data 163 is stored in memory for further processing by processing system 162, as disclosed herein. Thus, in the example of FIG. 3, the device (e.g., catheter) can include one or more electrical sensors, one or more EM sensors and one or more electrodes for delivering treatment or other signals to surrounding tissue in a contact or non-contact manner.

As a further example, the signal generator 161 supplies a localization signal to surrounding tissue in the patient's body 154 via one or more electrodes on the device 156. The localization signals can be measured by the non-invasive plurality of sensors 170 attached to the body 154 at locations in a three-dimensional coordinate system, such as determined by geometry calculator 180. The sensors 170 thus can sense electrical activity, including signals corresponding to the applied localization signals. As mentioned, the sensors 164 can also sense other electrical signals, such as corresponding to real-time electrograms for the patient's heart.

The placement of the device 156 can be guided via a localization method 194. As an example, the localization method 194 includes instructions executed by the processing system 162 to localize the device 156 employing an equivalent dipole model and measurements based on electrical measurement data 176 from the body surface sensors 170. For instance, the localization method 194 can compute a solution for a dipole model cost function to provide coordinates for the signal emitting element on the device to localize the device 156 and its electrodes, such as disclosed in U.S. Patent Publication No. 2016/0061599, entitled LOCALIZATION AND TRACKING OF AN OBJECT. In other examples, the localization method can be determine spatial coordinates for the device based on the position data that is provided for one or more EM sensors on the device 156. The guidance provided via the localization method 194 can be automated, semi-automated or be manually implemented based on information sensed. During localization, the electrode on the device 156 can contact or not contact the patient's heart 152, endocardially or epicardially.

By way of further example, localization method 194 can be implemented with respect to higher amplitude spikes or other signals that physician delivers via the device 156 for various clinical reasons. The localization results would presumably be quite confident in this localization because SNR is high. Immediately following the clinical higher amplitude signal, a localization signal (e.g., normal low output pulses that do not stimulate or achieve the same therapeutic effect) can be delivered via one or more electrodes on the device 156, and the localization method 194 can then compute the position of the device in response to the localization signal. The difference in position for each of the different types localizations can be computed, and the difference can be used to calibrate the system. For example, if catheter delivers high amplitude pacing pulse for another reason at a location A, dipole would localize to position A. Then with catheter remaining at location A' (the same or slightly displaced version of location A), another localization signal with lower output current can be applied, and localization engine can employ the dipole method to localize to location A'. The system would calibrate position A' as true position A, and apply such computed calibration, which is stored in memory, for future localizations. The dipole calibration can also be guided by intraprocedural imaging (e.g., x-ray fluoroscopy, ultrasound, CT or the like) and/or based position data 168 provided by the EM spatial measurement system to confirm the localized position matches the corresponding position determined via the other modality. The localized position determined by the localization method 194 can be co-registered to a common coordinate system with such other modality, such as the into which the geometry calculator provides the relevant geometry data (e.g., envelope geometry and electrode geometry).

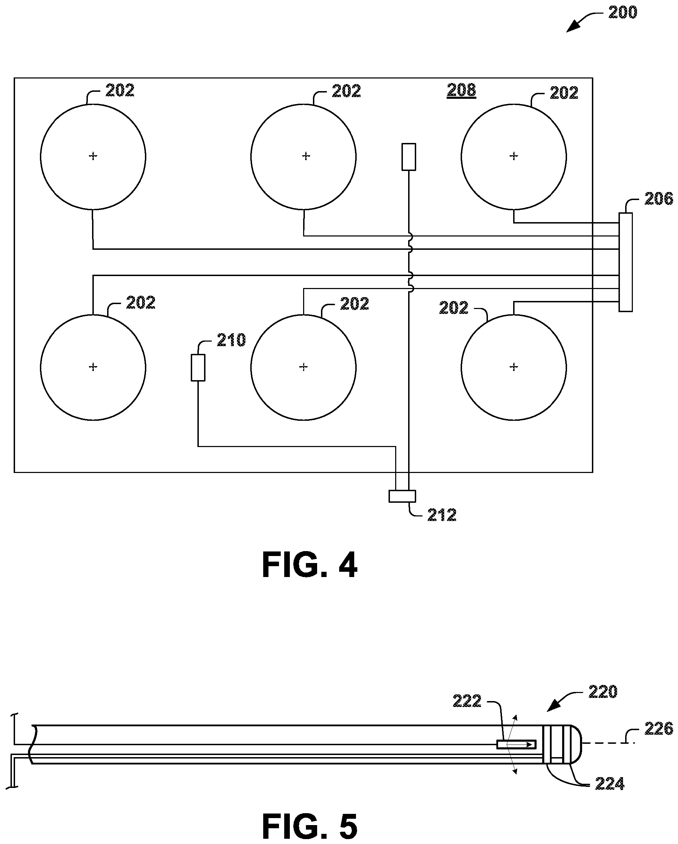

FIG. 4 depicts an example of a combined electrical and EM sensing device 200. The device 200 includes a plurality of electrodes 202 that can be utilized to sense electrical activity from an outer surface of the patient's skin and provide corresponding sensor signals indicative of the sensed electrical activity. The electrodes can correspond to contact or non-contact sensor electrodes to sense the electrical activity at the surface of the patient's body. Each of the sensor electrodes 202 can be electrically coupled (e.g., via conductive trace or electrical wires) to a terminal connector 206. The connector 206 can include an arrangement of electrodes and housing features to electrically connect each of the sensor electrodes 202 to a sensing interface apparatus that can include an arrangement of amplifiers and filters for providing the sensed electrical data (e.g., corresponding to electrical measurement system 14, 72, 172).

The device 200 can also include a substrate 208 to which each of the electrodes 202 can be attached. The substrate can be compliant and flexible to facilitate attachment to the body surface and accommodating the contours of such surface. The sensor device 200 can come in a variety of shapes and sizes that can be utilized to accommodate patients of different sizes. The electrical traces interconnecting the electrodes 202 and the terminal 206 can be embedded in the substrate 208, such as interposed between layers thereof or otherwise insulated and shielded to facilitate propagation of the electrical signals while mitigating interference with other signals.

The device 200 also includes a plurality of EM sensors 210. Each of the EM sensors 210 can include a sensor coil, such as a single sinusoidal coil that can provide five or six degree of freedom sensing for each EM sensor 210. Each EM sensor thus provides a sensor signal in response to current induced when exposed to an EM field, such as disclosed herein. The sensor signals can be provided via electrically conductive traces or wires from each respective sensors 210 along the surface of the substrate 208 and terminate in a corresponding terminal connector 212. Each of the electrical traces can be appropriately shield to mitigate interference among the electrical and EM sensor signals. The terminal connector 212 can be one of a mating or other connector that can couple the set of EM sensors 210 with a corresponding EM measurement device, such as disclosed herein.

In other examples, a wireless communication protocol can be used to propagate the EM sensor signals to a processing device (e.g., controller 21 of the EM spatial measurement system 12). For instance, the EM sensor can be implemented as a radio frequency identifier (RFID) transmitter that is activated to transmit RFID data and signal strength information in response to receiving the varying EM field or another interrogating radio frequency signal. Other wireless technologies could also be used to communicate the EM sensor signals.

Each of the electrodes 202 has a known position with respect to each other. For example, distance between centers of each of the respective electrodes 202 can be predetermined over the surface of the device 200. Each of the EM sensors 210 are similarly positioned at a predetermined spatial location with respect to each of the sensor electrodes 202. Thus, by knowing the spatial relationship (geometry) between each of the EM sensors 210 and the respective electrical sensors 202, the spatial coordinates of the electrical sensors can be determined readily from the position data representing the spatial coordinates of the EM sensors.

In the example of FIG. 4, the EM terminal connector 212 and the electrical sensor's terminal connector 206 are separate, such as to enable separate connections to an electrical measurement system and a corresponding EM measurement system. In other examples, a common set of terminal connectors can be implemented for propagating both EM sensor signals and electrical sensor signals to a common integrated control unit that is configured to process both EM sensor signals for determining position in three-dimensional space in which the EM field is provided and for processing the electrical sensor signals based upon the sensed electrical activity measured from the body surface via electrodes 202.

FIG. 5 depicts an example of a catheter 220 incorporating both one or more EM sensor 222 and one or more electrical sensor 224. The catheter 220 can be the probe device that is inserted into the body, such as device 56, 156 disclosed in FIGS. 1-3. Thus, in the example of FIG. 5, the EM sensor 222 includes an elongate form having a longitudinal Z-axis that is aligned and substantially parallel with the longitudinal axis 226 of the body of the catheter. As disclosed herein, the EM sensor 222 can be a five or six degree freedom sensor coil that can provide a corresponding sensor signal in response to residing within an EM field such as provided by a field generator (e.g., field generator 18). Other types of EM sensor coils may also be utilized and, in some examples, the catheter can include more than one EM sensor.

The location and orientation of the EM sensor 222 is known a priori with respect to each of the one or more electrical sensors 224 on the catheter body. For instance, the electrodes 224 can be implemented as annular rings circumscribing the distal end of the catheter body, spaced a predetermined distance from its tip. In this way, the location of each electrode 224 and the catheter tip, which may or may not include an electrode, can be determined based upon position data derived from the EM sensor signal that is produced by the sensor 222 in response to an EM field, such as disclosed herein.

As a further example, one or more of electrodes 224 can be utilized to supply an electrical signal such as can be provided by a signal generator external to the patient's body and provided to the electrode via an electrical conductor. Additionally or alternatively, one or more electrodes 224 are implemented as sensing electrodes to be positioned on or near a surface for contact or non-contact sensing of electrical activity within the patient's body. The one or more sensing electrodes 224 can provide electrical signals via electrical conductors (e.g., shielded cables or traces) contained within channels or mounted to the catheter. The sensed electrical signals from sensors 224 can be provided to electrical sensing circuitry that can be stored in memory for subsequent processing. Similarly, the induced electrical signals from EM sensor 222 is provided to EM sensing circuitry to determine position data (e.g., data 22, 68, 168) that can be stored in memory for subsequent processing, such as disclosed herein.

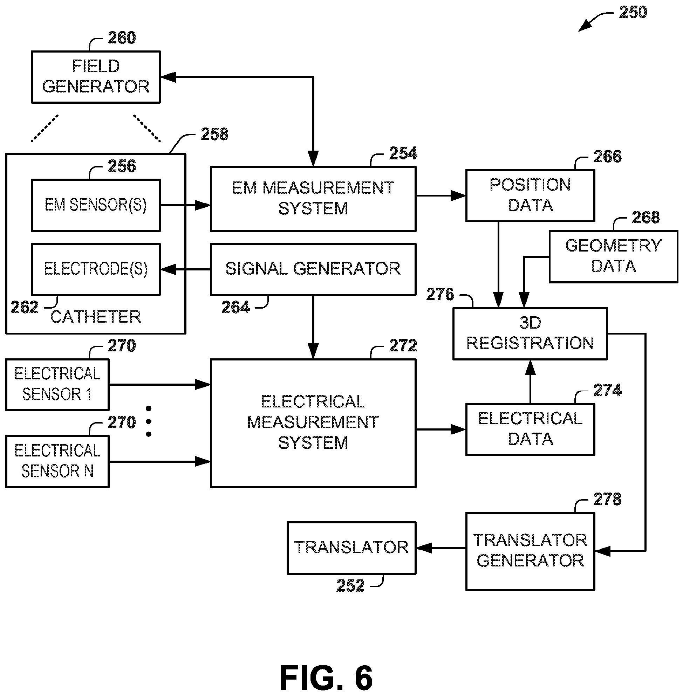

FIG. 6 depicts an example of a system 250 to generate a domain translator 252 to convert electrical data to corresponding position data in a three-dimensional coordinate system. Many of the components in this system have previously been introduced in other parts of this disclosure, but are being described in a particular approach to generate the spatial domain translator. Thus, the system 250 includes an EM measurement system 254 that receives sensor signals from one or more signals 256 such as may be carried by a catheter 258. For example, the catheter 258 can include one or more EM sensors 256 to provide respective sensor signals in response to an EM field generated by a field generator 260. The catheter 258 can also include one or more electrodes 262. As disclosed herein, each electrode 262 can be utilized to supply electrical signal to tissue in a contact or non contact manner in response to a stimulus signal provided by a signal generator 264. The signal generator 264 can be external to the patient's body, for example.

In response signals detected by the EM sensor 256, the EM measurement system 254 can generate a corresponding position data 266. The position data 266 can include control points, representing 3D spatial locations in the EM spatial domain, and associated timing information (e.g., time-stamps). The timing information (e.g., from signal generator 264) can be provided to an electrical measurement system 272 to synchronize measured electrical signals with respect to signals supplied via the signal generator 264 to the electrode 262. A plurality of body surface sensors 270 are demonstrated as electrical sensors 1 through electrical sensors N, where N is a positive integer greater than or equal to 2. Each of the electrical sensors 270 constitute non-invasive sensors to measure electrical activity from the patient's body surface, such as disclosed herein. Each sensor thus can supply electrical signals representing sensed electrical activity on the body surface to electrical measurement system 272. The electrical measurement system 272 includes associated circuitry to generate an electrical data 274 corresponding to the sensed electrical activity. The measurement system 272, for example includes circuitry such as amplifiers, filters, and the like, to provide corresponding electrical data based on the sensed electrical activity on the surface.

As one example, by moving the catheter 258 across a region of interest (e.g., a portion or an entire surface while the signal generator 264 activates, intermittently, to supply a calibration signal to the electrode 262 at a plurality of discrete locations across the region of interest, respective data sets 266 and 274 are generated in each of the EM-spatial domain and the electrical domain for tagging the location via the catheter 258. For instance, the electrical data 274 and the position data 266 both can be stamped with timing information (e.g., time stamp metadata) to synchronize position data 266 derived from the EM sensors with the measured electrical data 274. The system can also include a 3D surface geometry for the region of interest, such as a 3D surface mesh, which can be derived from position data 266, imaging data or another method. A 3D registration function 276 performs three-dimensional registration based on the position data 266, electrical data 274 and geometry data 268. That is, the 3D registration can temporally and spatially register the sensed electrical data (in the electrical domain) 274 from the sensors 270 with respect to the position data 266 (in the EM domain) at the time when the signal is supplied to the electrode. In this way, the electrical data measured for each calibration pulse can operate as a surrogate for the spatial coordinates known from the EM sensor 256.

In one example, the translator generator 278 generates the translator 252 based upon the correspondence between each EM signal that is generated for a given position of the EM sensor 256 and the sensed electrical data responsive to the generated signal for such position. The registration function 276 can create a correspondence between each magnetic sensor location, corresponding to control points in the EM domain, and the electrical signals detected by the sensors 270, such as are fixed on the body surface. In this way each set of electrical signals provided by the electrical data can The domain translator generator 278 can receive the correspondence information between the position data and electrical data for each tagged location and in turn generate the domain translator 252. For example, the domain translator 252 can be generated as a look-up table that maps directly between the set of electrical sensor signals across the body surface to a corresponding tagged position in a given coordinate system (e.g., coordinate system of the EM measurement system or other). The look-up table provides a direct mapping between each set of electrical signals and a respective EM location in which the set of electrical data operates as an index to the look-up table to provide the location. The resolution of the look-up table can be increased by increasing the number of control points on the surface of interest in the EM domain that are being mapped to electrical data sets. The matching of electrical signals can be implemented by calculating a similarity between a newly detected set of electrical signals (e.g., provided as electrical data 274), which set of signals is temporally synchronized with an signal applied to an electrode via the signal generator 264, and the electrical signals stored in the look-up table. For example, the similarity between input data set and the signals in the look-up table can be computed using a cross correlation to bypass signal magnitude difference; although other methods can be used to compute the similarity.

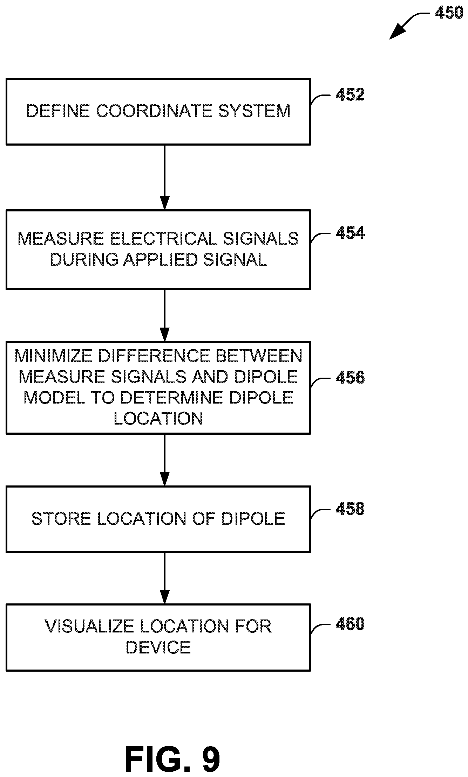

Alternatively or additionally, instead of the domain translator 278 being programmed as a look-up table between the magnetic sensor location and electrical sensor signals, the registration component 276 can utilize predetermined locations for each respective electrical sensor 270 in a given coordinate system. Thus, for each location that is detected and tagged based on an EM sensor 256, the sensor generates to 254 simultaneously generates a corresponding impulse or other supplied signal. The electrical signals from sensors 270 thus can record the sensor signal that is supplied by the electrode within the patient's body to detect the location of the catheter in the corresponding three-dimensional coordinate system to which the sensors have been registered. The location determined for the catheter 258 can thus create a correspondence between the magnetic sensor location and the location determined using the electrical signals (e.g., computed by a localization method, such as the dipole method of FIG. 9). Thus, by moving the catheter and respective sensors 256 and 262 across the surface of interest, plurality of locations can be tagged to create sets of geometry and a corresponding registration thereof via the registration method 276. The domain translator generator 278 can compute a transform T to convert from locations of the electrical based localized detection method to the locations of the EM based detection. The transform constituting the translator 252 can be utilized in subsequent applications.

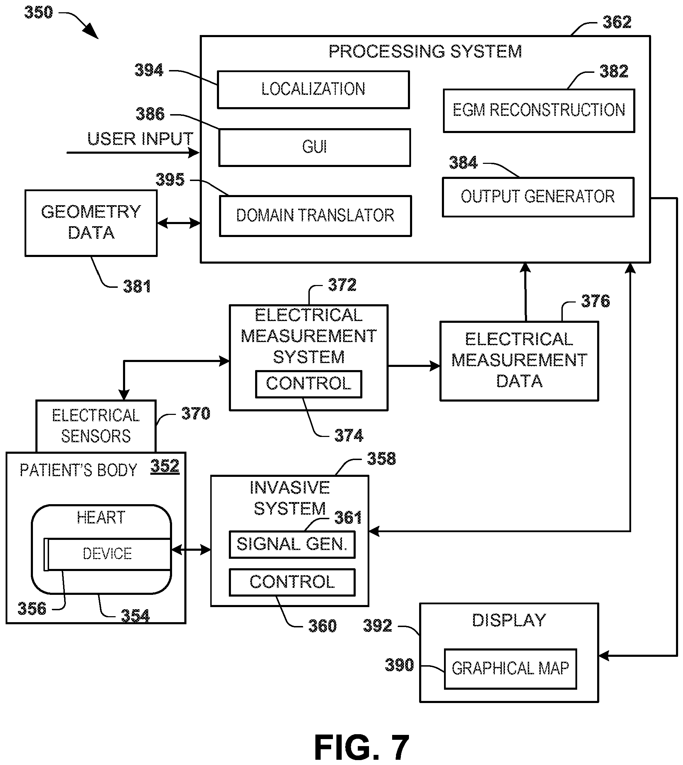

By way of example, FIG. 7 depicts another example of a system 350 to facilitate evaluation and treatment of cardiac function. The same or similar components to those introduced with respect to FIG. 2 are demonstrated in FIG. 7 using the same reference numbers increased by adding 300 (same as in FIG. 3 but increased by adding 200). Accordingly, reference can be made back to the description of FIGS. 2 or 3 for additional information about such common components.

The system 350 of FIG. 7 provides an approach that utilizes a domain translator 395 that is generated based on information collected using EM sensors. However, since not all catheters are or can be easily equipped with EM sensors, the translator 395 enables use of a catheter (e.g., for evaluation and/or treatment) not equipped with an EM sensor but that does include an electrode coupled to a signal generator for supplying an interrogation signal from a signal generator 361 as the catheter is navigated within the patient's body. The signal generator 361 can provide the interrogation signal as a localization signal, such as disclosed herein, which can be intermittently or periodically supplied to the electrode during navigation and/or in response to user input. The interrogation signal may also be correspond to energy supplied to the device 356 to deliver a therapy, such as disclosed herein. The translator 395 converts the electrical measurement data 376, which is generated based on body surface electrical activity measured via electrical sensors 370, to corresponding three-dimensional location in a given coordinate system. As disclosed herein, the translator 395 can be implemented as a LUT or a transform for example.

As also disclosed herein, geometry data 381 can be generated (e.g., by geometry calculator) based on EM sensor position data acquired for an anatomic envelope and electrode locations in the given coordinate system. The geometry data 381 thus can stored in memory to include both envelope geometry and electrode geometry to enable the electrogram reconstruction 382 to reconstruct electrical activity from the body surface onto the cardiac envelope based on electrical measurement data. In this way, once the geometry data 381 and translator 395 are generated, one can navigate around the catheter device 356 based on relative location of the catheter to these known geometries, and the translator can update the location during such navigation.

The system 350 can also include a localization method to determine spatial coordinates in a given domain. This may be in addition to or as an alternative to the location provided by the translator 395. For example, the localization method can implement a dipole localization method based on electrical measurement data from sensors 370 where the locations of the sensors are known, such as based on imaging, EM position measurements or the like.

The coordinates determined by the localization method 394 and/or by the domain translator 395 can be utilized by an output generator 384 to display location information represent for the device 356 in three-dimensional space based on coordinates determined according to one or more approaches herein. Additionally, the location (or a corresponding path) can be displayed at the spatial locations across a cardiac envelope (e.g., on an epicardial or endocardial surface of the heart 354). The output generator 384 can display the location separately. In other examples, the location can be combined with other output data, such as to display location information on graphical map of electrical activity of the heart 354. Additionally or alternatively, the localization can be continuous process and/or be synchronized with respect to the application of therapy provided by the system 358.

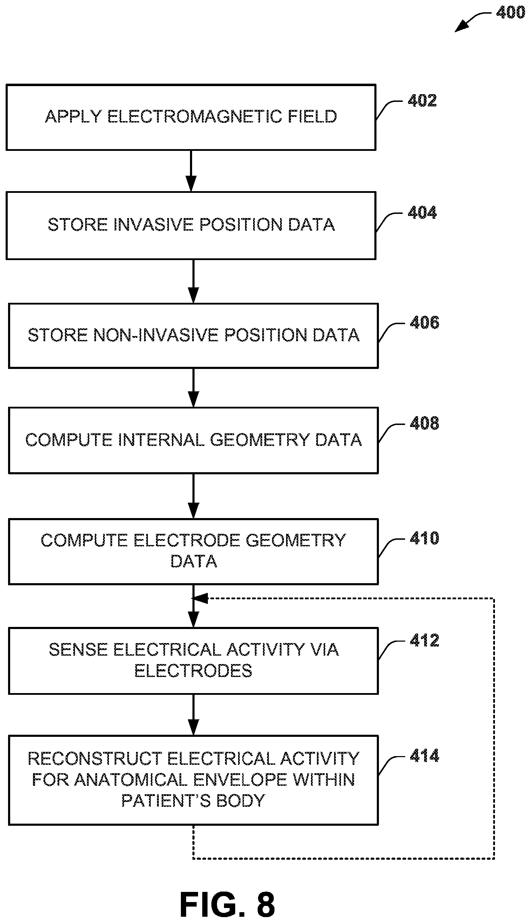

In view of the foregoing structural and functional features described above, example methods will be better appreciated with reference to FIGS. 8 and 9. While, for purposes of simplicity of explanation, the example methods are shown and described as executing serially, the present examples are not limited by the illustrated order, as some actions could, in other examples, occur in different orders. Moreover, it is not necessary that all described actions be performed to implement a method and other actions can be combined with those shown as disclosed herein. The example methods of FIG. 8 and 9 can be implemented as computer-readable instructions that can be stored in a non-transitory computer readable medium such as can be computer program product. The computer readable instructions corresponding to the methods can also be executed by a processor.

FIG. 8 is a flow diagram depicting an example of a method 400 for using EM sensing to generate geometry information. The method begins at 402 in the context of an EM field being applied (e.g., by field generator 18, 66, 166, 260). The field can be initiated in response to a user input or an automated trigger associated with the method 400. At 404, invasive position data is stored in memory. The invasive position data represents different positions of one or more sensors (e.g., EM sensor 20, 210, 222) in a given coordinate system within a volume defined by the electromagnetic field provided at 402.

At 406, non-invasive position data is stored. For instance, the non-invasive position data represents different positions of a plurality of control points (e.g., corresponding to EM sensors) in the given coordinate system associated with a position of one or more sensor electrodes (e.g., sensors 24, 70, 170, 202, 224 or 370). The control points can correspond to known locations of EM sensors that are spaced apart from sensor electrodes by a predetermined distance. In other examples, one or more of the control points can correspond to known locations of EM sensors that are co-located with the sensor electrodes. There can be a one EM sensor for each sensor electrode or there can be different number of the EM sensors and sensor electrodes (e.g., fewer EM sensors than sensor electrodes).

At 408, internal geometry data is computed (e.g., by geometry calculator 26, 80, 180). For instance, the internal geometry data is computed based on the invasive position data to represent a three-dimensional anatomical surface for a region of interest within a patient's body. The internal geometry data provide an anatomical model for the region of interest. For example, the internal geometry data can be a mesh model of the anatomical surface by describing a polygon mesh (e.g., a triangle mesh) of vertices and edges in the given 3D coordinate system. The mesh thus can include concave and convex polygons depending on the contours of the surface (e.g., endocardial and/or epicardial) that is modeled. At 410, electrode geometry data is computed (e.g., by geometry calculator 26, 80, 180). The electrode geometry data is computed based on the non-invasive position data to represent a location (e.g., point, such as a centroid) of each of a plurality of electrodes (e.g., sensors 24, 70, 170, 202 or 370) on an outer surface of the patient's body. The internal geometry and the electrode geometry can define geometry data in a common 3D coordinate system, such as for the EM spatial measurement system.

The geometry data thus can be stored in memory and used repeatedly to enable reconstruction of electrograms, as disclosed herein. The internal geometry data and electrode geometry data can be aggregated so that the location of each of a plurality of electrodes and the three-dimensional anatomical surface within a patient's body are in a common coordinate system to enable the reconstruction. The geometry data can be used for forward and inverse calculation of measured electrical signals, including electrical cardiac signals from heart, electrical signals from torso electrodes, and electrical signals from catheter, and combinations thereof.

For example, at 412, the electrical activity can be sensed from the plurality of electrodes (e.g., sensors 24, 70, 170, 202 or 370) on an outer surface of the patient's body. The electrical measurements can include signals during one or more time intervals. At 414 electrical activity sensed by the plurality of electrodes is reconstructed onto an anatomical envelope within the patient's body based on the internal geometry data and electrode geometry data. The reconstruction at 412 thus can be generated based on one or more intervals of body surface measured electrical activity, wherein the reconstructed electrical activity represents electrical activity across the three-dimensional anatomical surface within a patient's body during one or more such time interval. The time interval further may be selected in response to a user input.

As mentioned, by generating the geometry data using EM sensors, the cardiac electrical activity can be reconstructed on to the cardiac envelope. For example, using EM sensors on both catheter and body surface electrodes, one can detect locations of vest electrodes and catheter electrodes accurately in the absence of imaging. As noted, this can expedite the process (e.g., due to reduced computations) as well as decrease overall costs associated with the procedure. EM sensor(s) on a catheter thus can be used to create cardiac surface by moving the catheter around the region of interest, such as including LA, RA, LV, RV endocardial chambers. Since the EM fields are of a low field strength and can safely pass through human tissue, location measurement of an object is possible without the line-of-sight constraints of an optical spatial measurement system and without ionization of non-optical imaging modalities. The catheter can also be configured with electrodes or other delivery mechanisms to ablate tissue and to measure intracardiac signals.

FIG. 9 demonstrates an example of a localization method 450 that can be utilized (e.g., localization 194, 394). The method 450 can be used to localize an electrical field source within a patient's body based upon sensed electrical measurements at each of a plurality of known spatial locations. The field source can include one or more artificially applied fields, such as from an electrode on a fixed or moveable device (e.g., device 56, 156, 220, 356). In other examples the field source can correspond to biological signals, such as HIS bundle during QRS complex or pacing signals. Each such source can be represented a dipole model, having a dipole location and moment (direction and magnitude). The dipole model(s) can be adapted, via an optimization function, to determine the location of the field source based on measured electrical signals acquired in a non-invasive, invasive or hybrid non-invasive and invasive manner.

The method begins at 452 to define a spatial coordinate system. The coordinate system, for example, is a three-dimensional coordinate system that has been registered with respect to a patient's anatomy and/or an EM measurement system. The coordinate system can be derived from processing image data that includes sensors spatial locations as well as patient anatomy. The measurement system can include an arrangement of sensors (e.g., electrodes 24, 70, 170, 202, 224 or 370) distributed over a portion of the patient's torso surrounding the region in which the localization is to be performed. The locations of the sensors during localization can be the same or having a known spatial relationship with respect to the sensor locations in the image data used to define the coordinate system.

At 454, electrical signals are measured. The electrical signals are measured during a time that includes application of a localization signal. The localization signal can be a predetermined or otherwise deterministic signal that is applied at a site within a volume (e.g., patient's body) at an unknown location, which corresponds to the source being localized. The applied signals for dipole based localization can be any electrical signal including but not limited to pacing signals, or sinusoidal or square waves delivered to bipolar lead pairs from catheter.

There can be more than one such source that can apply respective signals. When more than one signal is applied at different sites being localized during the method 450, the signals can be differentiated. For instance, to detect multiple dipoles using pacing signals, the different lead pairs can be can be energized at different time instances to differentiate the signals corresponding to the different lead locations. To detect multiple dipoles using sinusoidal or square waves, such signals can be applied at different identifiable times (e.g., identified time stamps), concurrently at different frequencies or having other signal characteristics that can be identified individually. The measured electrical signals can be stored in memory as measurement data representing measured electrical signals at each of a plurality of known locations (e.g., measured by sensors at locations determined a priori) with respect to the given coordinate system (at 452).

The applied localization signal can be generated from one or more electrodes located at a position that is unknown and that is to be determined by the localization method 450. One or more electrodes can be disposed on a probe, such as a catheter or other device, at predetermined locations relative to each other. A signal generator can apply a specific signal that can be measured and localized by applying the dipole model for each electrode as disclosed herein. For example, the applied signal can be a predetermined waveform that is distinguishable from anatomical generated signals, such as may be a pulse, a sinusoidal waveform or the like that can be generated by a signal source electrically connected to the electrode(s) being localized. Extraneous signals (e.g., electrograms, noise or other signals) can be filtered out of the measurements, for example.

At 456, the method includes determining a dipole location and moment according to a predetermined optimization function for the dipole model. That is, the electrical signals measured at 454 provide known values that are used in the optimization function to find extrema that define the location and moment of the dipole with respect to the given coordinate system. As disclosed herein, the localization method 450 employs a dipole model cost function having parameters (variables) representing a dipole location and moment for localizing the source of the applied signal. The dipole location thus can be calculated as an optimization problem that fits the dipole model cost function to the data represented the measured electrical signals at each of the sensor locations, which are known in the coordinate system (at 452). In some examples, the determination at 456 can implement a boundary condition, as disclosed herein. Additionally or alternatively, the dipole model cost function can consider noise that exists in the electrode measurements (at 454).

At 458, the determined location and moment of the dipole can be stored in memory. The memory can include any local or remote memory (e.g., volatile and/or non-volatile memory) that is accessible for retrieval, such as for use by the same or different computer. The dipole location that is stored can thus represent spatial coordinates for the localized source. As mentioned, in some examples, there are multiple sources, which may be on the same or different probe, and respective locations can be stored at 458 for each such source.

At 460 the location for the source is visualized, such as in a display, printed output or the like (e.g., 92, 192 392). For example, the identified location can be overlaid in a graphical map of a patient's anatomy, such as a heart or other anatomic region where the source was localized to reside via the method. By determining the dipole location and graphical map in a common coordinate system or (via registration or transform) visualization of the dipole location, as well as a device carrying the field source for which the dipole location was determined at 456, is facilitated.

In the three dimensional coordinate system (defined at 452), the unknown parameters of the dipole model cost function determined at 456 can include the spatial coordinates for the 3D position of the dipole (r') and the dipole moment (p, having magnitude and direction). These unknown parameters can be computed as a function of the measurements and known spatial locations for such measurements. Additionally, since the dipole moment can be expressed in terms of the dipole location, as disclosed herein, the mathematical functions for the model can be expressed in terms of the unknown dipole location. Thus, the dipole model can parameterize the moving dipole as a vector having a dipole location parameter and a dipole moment parameter with respect to the given coordinate system. Further examples of dipole localization are disclosed in the
U.S. Patent Publication No. 2016/0061599.

In view of the foregoing structural and functional description, those skilled in the art will appreciate that portions of the invention may be embodied as a method, data processing system, or computer program product. Accordingly, these portions of the present invention may take the form of an entirely hardware embodiment, an entirely software embodiment, or an embodiment combining software and hardware. Furthermore, portions of the invention may be a computer program product on a computer-usable storage medium having computer readable program code on the medium. Any suitable computer-readable medium may be utilized including, but not limited to, static and dynamic storage devices, hard disks, optical storage devices, and magnetic storage devices.

Certain embodiments of the invention have also been described herein with reference to block illustrations of methods, systems, and computer program products. It will be understood that blocks of the illustrations, and combinations of blocks in the illustrations, can be implemented by computer-executable instructions. These computer-executable instructions may be provided to one or more processor of a general purpose computer, special purpose computer, or other programmable data processing apparatus (or a combination of devices and circuits) to produce a machine, such that the instructions, which execute via the processor, implement the functions specified in the block or blocks.

These computer-executable instructions may also be stored in computer-readable memory that can direct a computer or other programmable data processing apparatus to function in a particular manner, such that the instructions stored in the computer-readable memory result in an article of manufacture including instructions which implement the function specified in the flowchart block or blocks. The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide steps for implementing the functions specified in the flowchart block or blocks.

What have been described above are examples. It is, of course, not possible to describe every conceivable combination of components or methods, but one of ordinary skill in the art will recognize that many further combinations and permutations are possible. Accordingly, the invention is intended to embrace all such alterations, modifications, and variations that fall within the scope of this application, including the appended claims. Where the disclosure or claims recite "a," "an," "a first," or "another" element, or the equivalent thereof, it should be interpreted to include one or more than one such element, neither requiring nor excluding two or more such elements. As used herein, the term "includes" means includes but not limited to, the term "including" means including but not limited to. The term "based on" means based at least in part on."

## Claims

1. A system (10) comprising:
an electromagnetic spatial measurement apparatus comprising:
a moveable sensor (20) to provide a sensor signal in response to an electromagnetic field, the electromagnetic spatial measurement apparatus providing first position data representing multiple positions of the moveable sensor (20) in a given coordinate system;
a plurality of stationary sensors (24) configured to be positioned on an outer surface of a patient's body that each provides respective sensor signals in response to the electromagnetic field, the electromagnetic spatial measurement apparatus providing second position data based on a position of each of the plurality of stationary sensors (24) in the given coordinate system;
a plurality of electrodes (202) configured to be positioned on the outer surface of the patient's body to sense electrical activity from locations on the outer surface of the patient's body;
a processor configured to:
compute internal geometry data based on the first position data, the internal geometry data representing a plurality of locations in a three-dimensional space distributed across an anatomical surface within the patient's body;
compute electrode geometry data based on the second position data, the electrode geometry data representing the location of each of the plurality of electrodes (202) on the outer surface of the patient's body; and
reconstruct cardiac electrical activity onto a cardiac envelope based on the sensed electrical activity from the outer surface of the patient's body, the internal geometry data and the electrode geometry data.

2. The system of claim 1, wherein the processor further comprises instructions, corresponding to a localization engine, to determine an estimate of location of an applied signal in the given coordinate system based on the sensed electrical activity.

3. The system of claim 2, wherein the localization engine includes a domain translator (38) that comprises at least one of a look-up table or a transform that converts the sensed electrical activity for the plurality of electrodes (202) to an estimated location of the applied signal in the given coordinate system based on the sensed electrical activity by the plurality of electrodes (202).

4. The system of any of claims 1, 2 or 3, wherein the electromagnetic spatial measurement apparatus further comprises an electromagnetic field generator (18) to supply the electromagnetic field, and
wherein the system further comprises a probe device that is moveable within the electromagnetic field, wherein the moveable sensor is fixed with respect to the probe device.

5. The system of claim 4, wherein the electromagnetic spatial measurement apparatus further comprises an invasive electromagnetic sensor at a stationary position within the patient's body, the invasive electromagnetic sensor to provide a corresponding sensor signal in response to the electromagnetic field, the electromagnetic spatial measurement apparatus providing the invasive position data representing the stationary position of the invasive sensor in the given coordinate system based on the corresponding sensor signal.

6. The system of any of claims 1, 2, 3, 4 or 5, wherein the processor computes the internal geometry data to represent a plurality of locations in a three-dimensional space distributed across an anatomical surface within the patient's body.

7. The system of claim 6, wherein the processor computes the internal geometry data to represent a cardiac surface mesh.

8. The system of any of claims 1, 2, 3, 4, 5, 6 or 7, wherein the plurality of electrodes (202) are in a predetermined fixed relative spatial position with respect to the plurality of sensors (24), the processor employing position data determined for each of the plurality of stationary sensors (24) as control points to ascertain locations of each of the plurality of electrodes (202) in the given coordinate system.

9. The system of claim 8, wherein at least one of the plurality of stationary sensors (24) defines a respective control point in the given coordinate system that is co-located with a respective one of the plurality of electrodes (202).

10. The system of any of claims 1, 2, 3, 4, 5, 6, 7, 8 or 9, wherein at least one of the plurality of stationary sensors (24) is carried on a probe that is moveable to locations corresponding to locations of at least a substantial portion of the plurality of electrodes (202) or predetermined locations residing on a substrate (208) containing the plurality of electrodes (202) configured to be positioned on the outer surface of the patient's body.

11. The system of any of the preceding claims, further comprising:
a signal generator (61) to supply an electrical signal to an electrode on a probe device that is within a volume defined by the electromagnetic field;
an electrical measurement system (14) providing electrical measurement data representing electrical activity sensed by the plurality of electrodes (202) on the outer surface of the patient's body in response to the supplied electrical signal; and
an output generator (84) providing output data to a display (92) to provide an indication of a location of the electrode in the given coordinate system based on the electrical measurement data.

12. The system of claim 11, wherein the processor further comprises instructions, corresponding to a localization engine, to determine a location of an applied signal in the given coordinate system based on sensed electrical activity.

## Patentansprüche

1. System (10), umfassend:
eine elektromagnetische räumliche Messvorrichtung, umfassend:
einen bewegbaren Sensor (20), um ein Sensorsignal als Reaktion auf ein elektromagnetisches Feld bereitzustellen, wobei die elektromagnetische räumliche Messvorrichtung erste Positionsdaten bereitstellt, die mehrere Positionen des bewegbaren Sensors (20) in einem gegebenen Koordinatensystem repräsentieren;
mehrere stationäre Sensoren (24), die konfiguriert sind, um auf einer äußeren Oberfläche des Körpers eines Patienten positioniert zu werden, die jeweils entsprechende Sensorsignale als Reaktion auf das elektromagnetische Feld bereitstellen, wobei die elektromagnetische räumliche Messvorrichtung zweite Positionsdaten auf der Grundlage einer Position jedes der mehreren stationären Sensoren (24) in dem gegebenen Koordinatensystem bereitstellt;
mehrere Elektroden (202), die konfiguriert sind, um auf der Außenfläche des Körpers des Patienten positioniert zu werden, um die elektrische Aktivität von Stellen auf der Außenfläche des Körpers des Patienten abzutasten;
einen Prozessor, der für Folgendes konfiguriert ist:
Berechnen interner Geometriedaten basierend auf den ersten Stellendaten, wobei die internen Geometriedaten mehrere Stellen in einem dreidimensionalen Raum repräsentieren, die über eine anatomische Oberfläche innerhalb des Körpers des Patienten verteilt sind;
Berechnen von Elektroden-Geometriedaten basierend auf den zweiten Stellendaten, wobei die Elektroden-Geometriedaten die Stelle jeder der mehreren Elektroden (202) auf der Außenfläche des Körpers des Patienten repräsentieren; und
Rekonstruieren der elektrischen Herzaktivität auf einer Herzhülle basierend auf der gemessenen elektrischen Aktivität von der äußeren Körperoberfläche des Patienten, den internen Geometriedaten und den Elektroden-Geometriedaten.

2. System nach Anspruch 1, wobei der Prozessor ferner Befehle entsprechend einer Lokalisierungsmaschine umfasst, um eine Schätzung der Stelle eines angelegten Signals in dem gegebenen Koordinatensystem basierend auf der erfassten elektrischen Aktivität zu bestimmen.

3. System nach Anspruch 2, wobei die Lokalisierungsmaschine einen Domänenübersetzer (38) beinhaltet, der eine Nachschlagetabelle und/oder eine Transformation umfasst, die die erfasste elektrische Aktivität für die mehreren Elektroden (202) in eine Schätzung der Stelle des angelegten Signals in dem gegebenen Koordinatensystem basierend auf der erfassten elektrischen Aktivität durch die mehreren Elektroden (202) umwandelt.

4. System nach einem der Ansprüche 1, 2 oder 3, wobei die elektromagnetische räumliche Messvorrichtung ferner einen Elektromagnetfeldgenerator (18) umfasst, um das elektromagnetische Feld zu erzeugen, und wobei das System ferner ein Sondengerät umfasst, das innerhalb des elektromagnetischen Feldes bewegbar ist, wobei der bewegbare Sensor in Bezug auf das Sondengerät feststehend ist.

5. System nach Anspruch 4, wobei die elektromagnetische räumliche Messvorrichtung ferner einen invasiven elektromagnetischen Sensor an einer stationären Position innerhalb des Körpers des Patienten umfasst, wobei der invasive elektromagnetische Sensor in Reaktion auf das elektromagnetische Feld ein entsprechendes Sensorsignal bereitstellt, wobei die elektromagnetische räumliche Vorrichtung die invasiven Positionsdaten bereitstellt, die die stationäre Position des invasiven Sensors in dem gegebenen Koordinatensystem basierend auf dem entsprechenden Sensorsignal repräsentieren.

6. System nach einem der Ansprüche 1, 2, 3, 4 oder 5, wobei der Prozessor die internen Geometriedaten so berechnet, dass sie mehrere Stellen in einem dreidimensionalen Raum repräsentieren, die über eine anatomische Oberfläche innerhalb des Körpers des Patienten verteilt sind.

7. System nach Anspruch 6, wobei der Prozessor die internen Geometriedaten berechnet, um ein Herzoberflächennetz zu repräsentieren.

8. System nach einem der Ansprüche 1, 2, 3, 4, 5, 6 oder 7, wobei sich die mehreren Elektroden (202) in einer vorbestimmten festen relativen räumlichen Position in Bezug auf die mehreren Sensoren (24) befinden, wobei der Prozessor für jeden der mehreren stationären Sensoren (24) bestimmte Positionsdaten als Kontrollpunkte verwendet, um Stellen jeder der mehreren Elektroden (202) in dem gegebenen Koordinatensystem zu bestimmen.

9. System nach Anspruch 8, wobei mindestens einer der mehreren stationären Sensoren (24) einen jeweiligen Kontrollpunkt in dem gegebenen Koordinatensystem definiert, der zusammen mit einer jeweiligen Stelle der mehreren Elektroden (202) angeordnet ist.

10. System nach einem der Ansprüche 1, 2, 3, 4, 5, 6, 7, 8 oder 9, wobei mindestens einer der mehreren stationären Sensoren (24) auf einer Sonde getragen wird, die an Stellen bewegbar ist, die Stellen mindestens eines wesentlichen Abschnitts der mehreren Elektroden (202) oder vorbestimmten Stellen entsprechen, die sich auf einem Substrat (208) befinden, das die mehreren Elektroden (202) enthält, die konfiguriert sind, um auf der Außenfläche des Körpers des Patienten positioniert zu werden.

11. System nach einem der vorhergehenden Ansprüche, ferner umfassend:
einen Signalgenerator (61), um ein elektrisches Signal an eine Elektrode auf einem Sondengerät zu erzeugen, das sich innerhalb eines durch das elektromagnetische Feld definierten Volumens befindet;
ein elektrisches Messsystem (14), das elektrische Messdaten bereitstellt, die die elektrische Aktivität repräsentieren, die von mehreren Elektroden (202) an der Außenfläche des Körpers des Patienten als Reaktion auf das zugeführte elektrische Signal erfasst wird; und
einen Ausgabegenerator (84), der Ausgabedaten für eine Anzeige (92) erzeugt, um eine Angabe einer Stelle der Elektrode in dem gegebenen Koordinatensystem basierend auf den elektrischen Messdaten bereitzustellen.

12. System nach Anspruch 11, wobei der Prozessor ferner Befehle umfasst, die einer Lokalisierungsmaschine entsprechen, um eine Stelle eines angelegten Signals in dem gegebenen Koordinatensystem basierend auf der gemessenen elektrischen Aktivität zu bestimmen.

## Revendications

1. Système (10) comprenant :
un appareil de mesure spatiale électromagnétique comprenant :
un capteur mobile (20) destiné à fournir un signal de capteur en réponse à un champ électromagnétique, l'appareil de mesure spatiale électromagnétique fournissant des premières données de position représentant plusieurs positions du capteur mobile (20) dans un système de coordonnées donné ;
une pluralité de capteurs fixes (24) configurés pour être positionnés sur une surface externe du corps d'un patient qui fournissent chacun des signaux de capteur respectifs en réponse au champ électromagnétique, l'appareil de mesure spatiale électromagnétique fournissant des secondes données de position basées sur une position de chacun de la pluralité de capteurs fixes (24) dans le système de coordonnées donné ;
une pluralité d'électrodes (202) configurées pour être positionnées sur la surface externe du corps du patient pour détecter l'activité électrique à partir d'emplacements situés sur la surface externe du corps du patient ;
un processeur configuré pour :
calculer des données de géométrie interne sur la base des premières données de position, les données de géométrie interne représentant une pluralité d'emplacements dans un espace tridimensionnel réparti sur une surface anatomique à l'intérieur du corps du patient ;
calculer des données de géométrie d'électrode sur la base des secondes données de position, les données de géométrie d'électrode représentant l'emplacement de chacune de la pluralité d'électrodes (202) sur la surface externe du corps du patient ; et pour
reconstruire l'activité électrique cardiaque sur une enveloppe cardiaque sur la base de l'activité électrique détectée à partir de la surface externe du corps du patient, des données de géométrie interne et des données de géométrie d'électrode.

2. Système selon la revendication 1, dans lequel le processeur comprend en outre des instructions, correspondant à un moteur de localisation, pour déterminer une estimation de l'emplacement d'un signal appliqué dans le système de coordonnées donné sur la base de l'activité électrique détectée.

3. Système selon la revendication 2, dans lequel le moteur de localisation comprend un traducteur de domaine (38) qui comprend au moins une table de consultation ou une transformée qui convertit l'activité électrique détectée pour la pluralité d'électrodes (202) en un emplacement estimé du signal appliqué dans le système de coordonnées donné sur la base de l'activité électrique détectée par la pluralité d'électrodes (202).

4. Système selon l'une quelconque des revendications 1, 2 ou 3, dans lequel l'appareil de mesure spatiale électromagnétique comprend en outre un générateur de champ électromagnétique (18) pour fournir le champ électromagnétique, et le système comprenant en outre un dispositif de sonde qui est mobile dans le champ électromagnétique, dans lequel le capteur mobile est fixe par rapport au dispositif de sonde.

5. Système selon la revendication 4, dans lequel l'appareil de mesure spatiale électromagnétique comprend en outre un capteur électromagnétique invasif à une position fixe dans le corps du patient, le capteur électromagnétique invasif étant destiné à fournir un signal de capteur correspondant en réponse au champ électromagnétique, l'appareil de mesure spatiale électromagnétique fournissant les données de position invasive représentant la position fixe du capteur invasif dans le système de coordonnées donné sur la base du signal de capteur correspondant.

6. Système selon l'une quelconque des revendications 1, 2, 3, 4 ou 5, dans lequel le processeur calcule les données de géométrie interne pour représenter une pluralité d'emplacements dans un espace tridimensionnel réparti sur une surface anatomique à l'intérieur du corps du patient.

7. Système selon la revendication 6, dans lequel le processeur calcule les données de géométrie interne pour représenter un maillage de surface cardiaque.

8. Système selon l'une quelconque des revendications 1, 2, 3, 4, 5, 6 ou 7, dans lequel la pluralité d'électrodes (202) sont dans une position spatiale relative fixe prédéterminée par rapport à la pluralité de capteurs (24), le processeur employant des données de position déterminées pour chacun de la pluralité de capteurs fixes (24) en tant que points de commande pour déterminer les emplacements de chacune de la pluralité d'électrodes (202) dans le système de coordonnées donné.

9. Système selon la revendication 8, dans lequel au moins l'un de la pluralité de capteurs fixes (24) définit un point de commande respectif dans le système de coordonnées donné qui est co-localisé avec une électrode respective de la pluralité d'électrodes (202).

10. Système selon l'une quelconque des revendications 1, 2, 3, 4, 5, 6, 7, 8 ou 9, dans lequel au moins l'un de la pluralité de capteurs fixes (24) est porté sur une sonde qui est mobile vers des emplacements correspondant aux emplacements d'au moins une partie substantielle de la pluralité d'électrodes (202) ou des emplacements prédéterminés reposant sur un substrat (208) contenant la pluralité d'électrodes (202) configurées pour être positionnées sur la surface externe du corps du patient.

11. Système selon l'une quelconque des revendications précédentes, comprenant en outre :
un générateur de signal (61) destiné à fournir un signal électrique à une électrode sur un dispositif de sonde qui se trouve dans un volume défini par le champ électromagnétique ;
un système de mesure électrique (14) fournissant des données de mesure électrique représentant l'activité électrique détectée par la pluralité d'électrodes (202) sur la surface externe du corps du patient en réponse au signal électrique fourni ; et
un générateur de sortie (84) fournissant des données de sortie à un affichage (92) pour fournir une indication d'un emplacement de l'électrode dans le système de coordonnées donné sur la base des données de mesure électrique.

12. Système selon la revendication 11, dans lequel le processeur comprend en outre des instructions, correspondant à un moteur de localisation, pour déterminer un emplacement d'un signal appliqué dans le système de coordonnées donné sur la base d'une activité électrique détectée.
